# EUROPEAN PATENT SPECIFICATION

(11) **EP 1 071 818 B1**
(45) Date of publication and mention of the grant of the patent: **05.12.2007**
(21) Application number: 99916071.6
(22) Date of filing: 19.04.1999
(51) Int. Cl.: C12Q 1/68, C12Q 1/04

(54) **METHOD FOR THE RAPID DETERMINATION OF BACTERIA**
VERFAHREN ZUR SCHNELLEN BESTIMMUNG VON BAKTERIEN
METHODE RAPIDE DE DETERMINATION BACTERIENNE

(30) Priority: 20.04.1998 EP 98201253
(43) Date of publication of application: 31.01.2001
(73) Proprietor: ACADEMISCH ZIEKENHUIS GRONINGEN, 9713 EX Groningen (NL); Microscreen B.V., 9713 GX Groningen (NL)
(72) Inventor: JANSEN, Gijsbert, Johan, NL-9061 BJ Giekerk (NL); DEGENER, John, Edward, NL-9203 LP Drachten (NL); WELLING, Gjalt, Wietze, NL-9315 PC Roderwolde (NL)
(74) Representative: Prins, Adrianus Willem
(86) International application number: PCT/NL1999/000223
(87) International publication number: WO 1999/054502

(56) References cited:
- EP-A- 0 277 237
- EP-A- 0 479 117
- WO-A-93/24659
- WO-A-97/05282
- FR-A- 2 659 981

## Description

The invention relates to the detection, identification or determination of bacteria in samples in general and in particular in clinical samples such as blood, urine, saliva, cerebrospinial fluid, faeces, pus and tissue that are taken from patients that are possibly infected with a, as yet unknown, possibly pathogenic bacterium, or during follow-up diagnostic testing to for example evaluate therapeutic measures that have been taken so far to treat the disease.

Traditional methods to determine or identify bacteria in general start with a Gram-stain, which is well known in the art. Such a stain can be performed on a sample immediately after sampling or, when not enough bacteria are present, after a short period of culturing of the sample. In general, four types of bacteria are found after Gram-staining; Gram-negative rods and cocci and Gram-positive rods and cocci. However, such a Gram-stain can only in very exceptional cases provide the clinician with the knowledge required to provide accurate therapy.

Examples of Gram-negative rods in clinical samples are *Enterobacter, Klebsiella, Salmonella, Escherichia, Proteus* and *Pseudomonas species,* of Gram-negative cocci are *Neisseria species.* Gram-positive rods that may be found in clinical samples are *Bacillus species,* of Gram-positive cocci are *Enterococcus, Streptoccus* and *Staphylococcus species.* Some of these, such as *Streptoccus* and *Staphylococcus* can easily be further determined or distinguished from each other by their morphological characteristics. *Streptococci* (and *Enterococci)* are characterised by chain-like character of cocci that are linked to each other whereas *Staphylococci* accumulate in clumps. Most other species, such as *Enterococcus* and *Streptococcus species* cannot be distinguished by morphology alone. However, such relatively rough taxonomic distinction on a genus level cannot be considered satisfactory for clinical purposes and consequently further identification is required to establish proper medication. For example in the case of *Staphylococcus,* these bacteria need to be further distinguished based on their coagulase positive (*S. aureus*) or coagulase negative (*S. haemolyticus* and others) character because these two groups require different antibiotic therapy.

In general, the exact species involved is determined by culturing techniques. To fully determine the species of a bacterium present in a clinical sample the following steps are in general required:
- (1) Pre-culturing of the sample in order to amplify the number of bacteria to a level above the lower detection limits of step (2).
- (2) Culturing on selective and non-selective media.

These traditional methods are time consuming. On average, a regular diagnostic procedure takes at least a few hours (minimally 2) of pre-culturing followed by minimally 24 hours of culturing on selective and non-selective media. This implies that it takes at least 26 hours before the clinician obtains a diagnosis on which he or she can select appropriate antibiotics or base other further treatment.

This latency-period between the sampling of a patient and the final diagnosis in most cases is critical for the treatment and the speed-of-recovery of the patient. During this latency-period a patient is in general treated with broad-spectrum antibiotics. The antibiotic of choice is mainly determined by the "clinical eye" of the clinician.

By selecting a broad-spectrum antibiotic, such therapies are in general successful in the eradication of the pathogen but a serious side effect of this strategy is the fact that in most instances the normal microbial flora is affected also. This side-effect heav.ily decreases the patients defence against microbial invaders from the environment. Especially the lowering of the colonisation threshold of the gastro-intestinal tract may cause severe overgrowth by e.g. yeasts and fungi. The resulting secondary infection, or super-infection, in septicaemic patients who already suffer from a decreased immunity often leads to life-threatening situations.

Apart from the serious danger to the patient's health, wide-spectrum antibiotic therapy poses another threat. The repeated exposure of indigenous bacteria to antibiotics enhances the emergence of resistance against such an antibiotic. Especially when a resistance-gene is encoded on a plasmid, other (potential pathogenic) bacterial species may become resistant after the uptake of the plasmid. This latter scenario is considered to be a major problem in hospital epidemiology. It is therefor of paramount therapeutic and epidemiological importance to speed up the methodological procedures in the diagnosis of blood samples from for example septicaemic patients to be able to select specific antibiotic therapy designed for the specific pathogen found, thereby refraining from using broad-spectrum antibiotics.

Present techniques other than culturing, albeit in general specific when beforehand knowledge exists about the species involved, cannot be used with samples containing uncharacterised species, and do thus not fit the acute needs when speedy diagnosis is needed on uncharacterised patient material. In general, these methods are also too slow to meet the needs of the clinician in providing care to his or her patients. Most, for example, require isolation of nucleic acid, or amplification of nucleic acid, or both, before the actual testing can be performed. Also, in those cases where resistance of a pathogen to antibiotics has already occurred, it is of utmost importance to be able to rapidly identify the pathogen and rapidly select the antibiotic against which the pathogen is not resistant. To do this, the micro-organism would again have to be cultured, to determine its resistance pattern, thereby again introducing a lag in diagnosis.

In short, there is a need for fast and reliable diagnosis of bacteria, present in for example clinical samples that may replace or add to the currently used culturing techniques.

The invention provides a method for determining a bacterium suspected of being present in a sample as defined in claim 1. Said method comprises
a) testing said sample by Gram-staining and
b) testing said sample with a probe according to an *in situ* hybridisation protocol selected on the basis of the outcome of said Gram-staining.

Rapid techniques for detecting bacteria and other bacteria in general are known. For example, *in situ* hybridisation is a well known technique, however, in general it has only been applied in specialised laboratories as a tool to detect and quantify the relative abundance of bacteria that are difficult to culture using traditional methodology or as a tool to quantify for example growth kinetics of already known bacteria in culture.

In short, in *in situ* hybridisation, nucleic acid probes, labelled with a reporter molecule such as an enzyme or a fluorescing substance, are reacted with specific nucleic acid sequences found specifically and preferably solely in the bacteria under study, which for this purpose has been permeabilised to let the probe enter the organism. As a target sequence nucleic acids of different origins are employed. Most commonly used as a target for *in situ* hybridisation is the 16S ribosomal RNA molecule. Other target molecules which can be used are: 23S ribosomal RNA, mitochondrial RNA, messenger RNA and nuclear DNA.

*In situ* hybridisation has never been successfully applied for rapid detection of bacteria in clinical samples because the presently used *in situ* hybridisation techniques are too inaccurate and too slow to give an advantage over traditional culturing.

First of all, hybridisation requires permeabilisation of the pathogen, and until now no generally applicable permeabilisation protocols have been developed that allow sufficient but restricted lysis of many or all of a broad range of unidentified bacteria. In general, mild permeabilisation leaves many bacteria (such as *Staphylococcus spec.)* inaccessible for subsequent hybridisation with probes, whereas rigorous permeabilisation often fully lyses most bacteria, thereby foregoing the possibility to detect them all together.

In addition, current protocols are in general time-consuming multi-step procedures; hybridisation often requires minimally 24 hours, thereby giving no relief to the needs of the clinician who is only helped with accurate and speedy diagnosis. Furthermore, they mostly require beforehand knowledge about the genus or even species involved in order to select appropriate probes; having such beforehand knowledge is clearly not the case in the event of a patient having an unidentified infection. Also, the present, already inappropriate hybridisation techniques do not allow to gather information on the response against antibiotics of the bacterium involved.

The invention provides a fast and reliable method for diagnosis, detection and/or determination of bacteria which may be present in a sample. Such a sample may be of various origin, it is for instance possible to apply a method as provided by the invention to a sample obtained from a (contaminated) bacterial culture, or drinking water or food suspected to be contaminated with a bacterium.

In a preferred embodiment the invention provides a method to detect or identify a bacterium suspected of being present in a clinical sample. Herein, the term "clinical sample" comprises a sample obtained or derived from an animal, preferably a mammal, more preferably a human being. Such a sample may be sampled or tested because a bacterial infection or disease is suspected. Such a sample can be of various origin, such as blood, serum, white blood cells, cerebrospinal fluid, synovial fluid, tissue, biopsies, urine, saliva, faeces, and others. In a preferred embodiment the invention provides a method wherein said sample is mammalian blood, preferably being derived from a human.

A sample can be a primary sample or it can be a secondary or sub-sample which is derived from a primary sample by diluting, splitting or culturing it one or more times. Diluting allows determining the relative abundance of a bacterium in a sample, thereby thus providing a method allowing not only qualitative but also quantitative determination of a bacterium. A sample can be tested directly after it has been obtained or after it has been stored, for example by cooling or freezing and secondary or sub-samples can be tested in parallel or subsequent from each other.

The invention provides a method comprising determining by Gram-staining the Gram-positive or Gram-negative and rod or coccus type of bacterium in a clinical sample and further testing said sample according to an *in situ* hybridisation protocol selected on the basis of the outcome of said Gram-staining. A primary advantage of a method according to the invention is the speed with which analyses can now be performed. Total analysis time, when using a method according to the invention, is greatly reduced, when compared with existing *in situ* hybridisation protocols. For example, Gram-positive Streptococci can now be determined from within about 30-60 minutes, Gram-negative rods from within about 45-90 minutes, if needed, whereas traditional protocols often need a working day or more. On top of that, often a first indication, or even a definitive selection, of a preferred antibiotic for therapy can be given, based on the results of the testing.

A preferred embodiment of the invention is a method for the detection or identification of bacteria in a clinical sample of blood of patients who are suspected to suffer from a septicaemia. In a preferred embodiment a method provided by the invention makes use of labeled probes, such as fluorescently labeled single strain DNA-, RNA- or PNA-probes, directed against specific target sequences on for example the ribosomal RNA of the target bacterium present in the sample.

The invention provides a method wherein classical Gram-staining indicates the presence of a Gram-negative or Gram-positive bacterium in said sample, further comprising determining the rod or coccus character of said bacterium, thereby establishing the subsequent testing protocol.

When a Gran-negative bacterium is of the rod type, the invention provides a method further comprising hybridising said sample with at least one probe selected from a group of probes capable of hybridising with nucleic acid found in *Escherichia coli,* in *Klebsiella pneumoniae,* in *Klebsiella oxytoca,* in *Serratia marcescens,* in *Enterobacter aerogenes,* in *Enterobacter cloacae,* in *Proteus vulgaris,* in *Proteus mirabilis,* in *Salmonella typhi,* in *Pseudomonas aeruginosa.*

Furthermore, the invention provides a method wherein said character is of the Gram-negative coccus type, further comprising subjecting said sample to treatment with a lysis buffer comprising lysozyme. Also, when said Gram-staining indicates the presence of a Gram-positive bacterium in said sample, said method is further comprising determining the rod or coccus character of said bacterium, and when said Gram-positve character is of the rod type, further comprising subjecting said sample to treatment with a lysis buffer comprising lysozyme and/or Proteinase K.

In addition, when said character is of the Gram-positive coccus type, a method is provided further comprising determining a chain-like or clump-like character of said bacteria before a hybridisation protocol is selected. When before mentioned character is chain-like, a method provided by the invention is further comprising subjecting said sample to treatment with a lysis buffer comprising lysozyme, and further comprising hybridising said sample with at least one probe selected from a group of probes capable of hybridising with nucleic acid found in *Enterococcus faecalis,* in *Streptococcus pneumoniae*, in *Streptococcus* mitis, in *Streptococcus viridans,* in *Streptococcus sanguis,* in *Enterococcus faecium.*

In addition, a method is provided wherein said character is clump-like, further comprising subjecting said sample to treatment with a lysis buffer comprising lysostaphin and or Proteinase K, further comprising hybridising said sample with at least one probe selected from a group of probes capable of hybridising with nucleic acid found in *Staphylococcus* aureus, in *Staphylococcus haemolyticus,* in *Staphylococcus saprophyticus.*

Probes used in a method as provided by the invention can be directed against various target nucleic acid molecules found in a bacterium which can be used are for example ribosomal RNA, mitochondrial RNA, plasmid DNA, messenger RNA and nuclear DNA. It is also possible to select as target molecules nucleic acid from the above discussed antibiotics resistance genes, which can be found in a plasmid or integrated in the bacterial genome.

In a preferred embodiment, a method provided by the invention uses as a target for *in situ* hybridisation a (16S) ribosomal RNA molecule. In a particular embodiment of the invention said probe is having no more than five, preferably no more than two mismatches with a probe selected of a group composed of probes having a sequence GCCTGCCAGTTTCGAATG or GTAGCCCTACTCGTAAGG or GAGCAAAGGTATTAACTTTACTCCC or GTTAGCCGTCCCTTTCTGG.or TTATCCCCCTCTGATGGG or AGAGAAGCAAGCTTCTCGTCCG or GCCACTCCTCTTTTTCCGG or GCTAATGCAGCGCGGATCC or CCGAAGGGGAAGGCTCTA or AGAGAAGCAAGCTTCTCGTCCGTT, each selected in relation to a method as provided by the invention or in relation to congruent antibiotic sensitivity of a bacterium recognised by said probe.

In addition, a method is provided by the invention that is further comprising hybridising said sample with at least one positive control probe capable of hybridising with nucleic acid found in a majority of bacterial species and/or with at least one negative control probe not being capable of hybridising with nucleic acid found in a majority of bacterial species. Preferably said majority comprises at least 90% of bacterial species, especially with those species found in general with possibly infected (septicaemic) patients. A method as provided by the invention is even more specific and/or sensitive when at least 95%, preferably at least 99% of said species is reactive with said positive control probe or no more than 5%, preferably no more than 1% is reactive with said negative control probe.

Such a positive or negative control probe as provided by the invention is given in the experimental part, in general said positive control probe comprises no more than five mismatches with a probe with the sequence GCTGCCTCCCGTAGGAGT and/or said negative control probe comprises no more than five mismatches with a probe with the sequence ACTCCTACGGGAGGCAGC.

Furthermore, the invention provides a method with additional value to the clinician in that in said method a probe is selected for its reactivity with one or a group of bacterial genera and/or (sub)species having congruent susceptibility to antibiotic treatment. Such a probe detecting or identifying a bacterium in a sample, preferably a clinical sample, is capable of hybridising with nucleic acid found in a group of bacterial genera and/or species or subspecies such as found with Staphylococcus and many other bacteria having congruent susceptibility to antibiotic treatment.

In a preferred embodiment of the invention, such a probe is having no more than five, preferably no more than two mismatches with a probe selected of a group composed of probes having a sequence GCCTGCCAGTTTCGAATG or GAGCAAAGGTATTAACTTTACTCCC (i.e. reactive with bacteria for which amoxycillin treatment is most likely effective) or GTAGCCCTACTCGTAAGG (cephalosporin treatment) or GTTAGCCGTCCCTTTCTGG (piperacillin and/or aminoglycoside) or TTATCCCCCTCTGATGGG or GCCACTCCTCTTTTTCCGG (amoxycillin) or GCTAATGCAGCGCGGATCC or CCGAAGGGGAAGGCTCTA (vancomycin)or AGAGAAGCAAGCTTCTCGTCCGTT or AGAGAAGCAAGCTTCTCGTCCG (flucloxacillin).

In a much preferred embodiment of the invention a one-step procedure is used for both binding target bacteria (in the sample) to a microscopic slide and fixing intracellular structures. In the experimental part, various lysis buffers and fixating technique are provided that utilise such a one-step procedure.

Furthermore, the invention provides a diagnostic test kit comprising means for detecting or identifying a bacterium suspected of being present in a sample using a method according to the invention or using a probe according to the invention. Such a diagnostic kit for example at least comprises probes or a set of probes specific for the detection of pathogenic bacteria. Instructions for a method comprising in situ hybridisation may be added. Optionally, said probes, which can be common nucleic acid or peptide nucleic acid probes, are linked to reporter molecules such as direct fluorescent labels. Other reporter molecules, such as enzymes or radioactive labels are also known.

In addition, said kit may comprise one or more of the necessary buffer solutions, such as lysis buffer or hybridisation, optionally in ready made form, or for example cover slips and reaction vials. Said kit may fully comprise sets of probes reactive with a wide gamut of (pathogenic) bacteria, optionally characterised by reactivity with bacteria of congruent antibiotic susceptibility, or may comprise sets of probes specifically directed against bacteria of Gram-positve of -negative, rod, coccus or chain- or clump-like character.

Such a kit may also comprise probes specifically reactive with antibiotic resistance genes, providing a positive identification of least applicable antibiotic treatment.

The invention is further explained in the experimental part of the description which is not limiting the invention.

### Experimental part

An example of a set of probes specific for the detection of pathogenic bacteria and an example of a new protocol for high-speed in situ hybridisation are presented. The methodology described here is for example used for both a preliminary screening of samples from septicaemic patients or as a full substitute on the basis of which therapeutic decisions are made. The invention thus provides a rapid and reliable method for determining the species and/or the genus of a bacterium present in a blood sample collected from a septicaemic patient.

### Components

A set of fluorescently labeled oligonucleotide probes designed to hybridise specifically with a group of pathogenic bacteria (i.e. *genus*-specific probe) or with one specific pathogen (i.e. *species*-specific probe) or with bacteria with congruent susceptiblity or resistance to antibiotics.

A protocol for fast *in situ* hybridization of bacteria present in samples of blood collected from septicaemic patients, using the said probes.

Oligonucleotide probes designed to hybridize specifically with a group of pathogenic bacteria.

In a particular embodiment of the invention a method provided by the invention is exemplified by making use of 16S rRNA target molecule because a large databank containing 16S rRNA-sequences exists and is freely accessible via the Internet. Labeled probes form an essential part in *in situ* hybridizations. The present invention provides a set of probes which have been designed in an unexpected novel manner i.e. not based on normal taxonomic principles but rather on their pathological significance. The group of probes which apply to this particular embodiment of the invention have been designed in such a way that they span group(s) of bacteria which are clustered on the basis of presumed congruent sensitivity to antimicrobial agents. Positive identification thus yields direct therapeutic information. Grouping bacteria on the basis of their presumed antibiotic susceptibility results in groups of bacteria containing one to several hundreds of different species. Although traditional methods can be used to detect the same set(s) of bacteria ,the use of a set of probes based on the criteria of presumed antibiotic susceptibility patterns is much faster over classical culturing methods that still suffer from fenotypic variability induced by environmental factors. The probes are preferably labelled with enzymatic or fluorescent labels. Current fluorescent labels which are applicable in this invention are:
1) Direct fluorescent labels:
   - Fluorescein-isothiocyanate (FITC)
   - Tetramethylrhodamine-5-isothiocyanate (TRITC)
   - Texasred™
   - 5(6)-carboxyfluorescein-N-hydroxysuccimide-ester (FLUOS™)
   - 7-amino-4-methylcoumarin-3-acetic acid (AMCA™)
   - Phycoerythrin
   - Indocarbocyanine dyes such as Cy3™, Cy5™ and C7™
   - Any other direct fluorescent label
2) Indirect fluorescent labels:
   - Enzymes such as alkaline phosphatase or horseradishperoxidase either attached directly or via a C6-thiol linker and used in combination with chemiluminescent substrates like AMPPD (3-('spiroadmantane)-4-methoxy-4-(3'-phosphoryloxy)-phenyl-1,2-dioxethane) or fluorescence generating substrates.
   - Digoxigenin (DIG) in combination with anti-DIG antibodies labeled with:
   - gold particles
   - fluorescent labels
   - Enzymes such as alkaline phosphatase or horseradish peroxidase, optionally in combination with chemiluminescent substrates like AMPPD (3-('spiroadmantane)-4-methoxy-4-(3'-phospho- ryloxy)-phenyl-1,2-dioxethane) or fluorescence generating substrates.
   - Biotin in combination with streptavidin or avidin and labeled just like the anti-DIG antibodies
   - Dinitrophenyl as hapten in combination with appropriate antibodies and labeled just like the anti-DIG antibodies
   - Any other indirect fluorescent or enzymatic label

Fluorescent labels allow direct microscopic analysis preferably combined with image analysis. For the detection of fluorescent oligonucleotide probes hybridised to ribosomal RNA of the target bacterium, photography can be applied. However quantitation by this method is hampered by the absence of objective criteria by means of which discrimination between hybridized and non-hybridized cells can be performed. Therefore for objective evaluation of probe-specificity, an image analysis system is employed which allows fluorimetrical reading of individual bacterial cells.

A protocol for fast *in situ* hybridization of bacteria present in samples of blood

Protocols for the detection of rRNA *in situ* typically utilise both a lytic reagent for permeabilisation of the bacterial cell wall and fixatives to preserve structural and molecular integrity of cellular components. However, the results of such hybridizations are highly dependent on the type, concentration and incubation-time of both the lytic reagent and the fixative. Component 2 of the invention consists of a protocol for *in situ* hybridization in which both permeabilization and fixation have been optimized for a subsequent hybridization of maximally 2 hours. For this protocol it was important to ensure that the hybridization procedure used was applicable to a wide variety of unidentified bacteria. Differentiated use of lytic reagents could only be based on information obtained from direct Gram-staining of the (pre-cultured) blood sample. After extensive validation the novel protocol had the following unique characteristics:

A optimal lytic reagent can be chosen on the basis of the gram-stain of the pathogen present in the sample of blood. This procedure of differentiated permeabilisation is novel to regular protocols for *in situ* hybridisation in which the permeabilisation is always dedicated to the permeabilisation of one or a defined group of target bacteria. In this new procedure a very wide array of unidentified bacteria can sufficiently be permeabilised without destruction of intracellular structures.

A one-step procedure is used for both binding target bacteria (in the sample) to the microscopic slide and fixing intracellular structures. Procedures presented in the current scientific literature all use multi-step protocols for binding, fixing and dehydration of the bacterial cells in order to condition them for optimal hybridisation.

The hybridisation time is shortened to 2 hours. Regular protocols for *in situ* hybridisation utilise a hybridisation time of minimally 24 hours, rendering them useless for rapid diagnostic applications.

The invention also provides kits for carrying out the rapid detection of bacteria in blood samples according to the invention. Such a kit will usually comprise at least a probe or probes and optionally other reagents such as components for hybridisation-fluid, washing-fluid and permeabilisation-fluid.

Such a kit may be applied in a routine bacteriology laboratory or in a bedside environment, both as a fast screening method or as a full substitute for classical identification methods.

### Examples of probe design and development

The following probes for example were found to hybridise the most predominant species of pathogens which are found in blood from septicaemic patients. In addition each probe hybridises with a species or a cluster of bacteria which share congruent (but often not identical) antibiotic sensitivity patterns

| ID Sequence (5'-3')¹ | Region² | Specificity³ |
|---|---|---|
| A GCTGCCTCCCGTAGGAGT | V2 | Bacterial Kindom |
| B ACTCCTACGGGAGGCAGC | n.d. | no matches |
| C GCCTGCCAGTTTCGAATG | V2 | Salmonella spp, Klebsiella spp, Enterobacter spp. |
| D GTAGCCCTACTCGTAAGG | V7 | K. oxytoca, S. marcescens, |
| | | Enterobacter spp, Proteus spp |
| E GAGCAAAGGTATTAACTTTACTCCC | V3 | E. coli |
| F TTATCCCCCTCTGATGGG | V2 | E. faecalis |
| G GCTAATGCAGCGCGGATCC | V2 | S. aureus, S. haemolyticus |
| H CCGAAGGGGAAGGCTCTA | V6 | S. aureus, S. saprophyticus |
| I AGAGAAGCAAGCTTCTCGTCCG | V1 | Streptococcus spp. |
| J GTTAGCCGTCCCTTTCTGG | V3 | P. aeruginosa |
| K AGAGAAGCAAGCTTCTCGTCCGTT | V2 | S. aureus |
| L GCCACTCCTCTTTTTCCGG | ?? | Enterococcus faecium |

| | | |
|---|---|---|
| ¹ Each probe optionally contains an FITC-label at the 5'-end ² The variable region on the 16S rRNA where the target-sequence of the probe is positioned. ³ The species or genus which rRNA contains a match with the sequence of the probe. | | |

### Protocol example.

A newly devised protocol for fast in-situ hybridization of pathogens in blood from septicaemic patients. This version consists of a step-wise version which can directly be used in a laboratory environment.
1 Collect a sample of blood from a patient using a vacuum sealed culture bottle.
2 Place the culture bottle in the pre-culturing machine (e.g. BactAlert, Organon Teknika, Durham, NC 27704) to monitor the growth of the pathogen. On-line monitoring is performed by measuring the pH of the sample.
3 After bacterial growth in a sample of blood has been detected, perform a Gram-stain and take out the culture bottle and collect 1 ml. of blood from the bottle using a syringe.
4 Using the syringe, put +/- 0.1 ml of this sample on a degreased glass slide. And streak out using a slide of glass.
5 Dry the slide for 5 minutes on a hotplate (for example of approximately 50°C).
6 Fix during 5 min. in ethanol(96%):formaldehyde(37%) (9:1.)
7 Dry the slide for 5 minutes on a hotplate. (Slides can be stored for several months if kept at room temperature in a dry chamber)
8 Permeabilise **Streptococci** 20 min at 25 C with lysozyme (1 g/l)
9 Permeabilise **Staphylococci** 20 min at 25 C with Lysostaphin (100 units/ml)
10 Rinse the slide with (demineralised) water for 5 minutes
11 Dry the slide for 5 minutes on a hotplate.
12 Pipet hybridisation buffer(+SDS)-probe mix ([probe]=10ng/µl). Cover with a coverslip.
13 Hybridize 2 hours (for example at 48°C).
14 Rinse 5 min using hybridisation buffer(-SDS).
15 Mount the slide with a coverslip.
16 Evaluate the slide.

### phosphate buffered saline

- 8 g/l NaCl
- 0.2 g/l KCl
- 1.44 g/l Na₂HPO₄
- 0.24 g/l KH₂PO₄
- adjust to pH 7.4.

### Hybridisation buffer (+SDS)

- 900 ml Milli-Q water
- 52.6 g NaCl
- 2.52 g Tris (hydroxymethyl)-aminomethane
- adjust to pH 7.5
- add 90 ml water.
- sterilize 15 minutes
- 10 ml SDS (10%) stock

### hybridisation buffer (-SDS)

- 900 ml Milli-Q water
- 52,6 g NaCl
- 2,52 g Tris
- adjust to pH 7.5
- 100 ml Milli-Q water
- sterilize 15 minutes

### hybridisation buffer-probe mix

- 10 ng/ul of lyophilized probe in hybridisation buffer

### lysozyme buffer

- 1,2 g Tris (=100mM)
- 1,86 g EDTA (=50mM)
- add 100 ml of milli-Q
- adjust to pH 7.5
- 0,05 to 0,2 g lysozyme

### Lysostaphin buffer

- 1,2 g Tris (=100mM)
- 1,86 g EDTA (=50mM)
- add 100 ml of milli-Q
- adjust to pH 7.5
- 0,05 to 0,2 g lysostaphin
- dilute a lysostaphin stock (1000 ug/ml in milli-Q, stored at -20°C) 5-20 times in the above mentioned buffer.

Optionally, to lyozyme buffer or lyostaphin buffer 0,05 to 0,2 mg/ml Proteinase K is added.
ethanol-formaldehyde (90:10)
- 1 ml formaldehyde 37%
- 9 ml ethanol 96%

### Validation of probe specificity

Specificity of probes was tested against the complete RDA-database (http://rdpwww.life.uiuc.edu:80/rdphome.html) of 15 august 1996 using the CheckProbe command and was considered sufficient if a no more than five, preferably no more than two mismatches were observed. Furthermore, to determine whether the probes could reach their specific target sequence, a reference collection of 20 of the most predominant bacteria in sepsis were hybridised using both the protocol and the probes mentioned here above. The result of this validation is listed in table 2. As can be read from this table all probes yield a satisfying hybridisation profile. Using the group-probes C and D it is possible to distinguish between: four groups of gram-negative rods:
C-positive and D-positive: Klebsiella oxytoca,
Enterobacter cloacae and Enterobacter aerogenes
C-positive and D-negative: Klebsiella pneumoniae and Salmonella typhi
C-negative and D-positive: Serratia marcescens and Proteus vulgaris
C-negative and D-negative: Proteus mirabilis.

For Escherichia coli and Pseudomonas aeruginosa two *species*-specific probes (E and J) have been designed and validated. These probes are optionally included because both Escherichia and Pseudomonas are notorious pathogens which demand specific antimicrobial therapy. Probe F is a species-specific probe for Enterococcus faecalis, a notorious pathogen. Probe I is a genus-specific probe which can be used in conjunction with probe F because Streptococci and Enterococci share the same morphology, while they require different antimicrobial treatment. Using both probes G and H, 4 separate species of Staphylococci can be distinguished:
G-positive and H-positive: Staphylococcus aureus
G-positive and H-negative: Staphylococcus haemolyticus
G-negative and H-positive: Staphylococcus saprophyticus
G-negative and H-negative: Staphylococcus epidermidis
Probe K is a *species*-specific-probe for Staphylococcus aureus and can be used to support the results obtained by probes G and H.

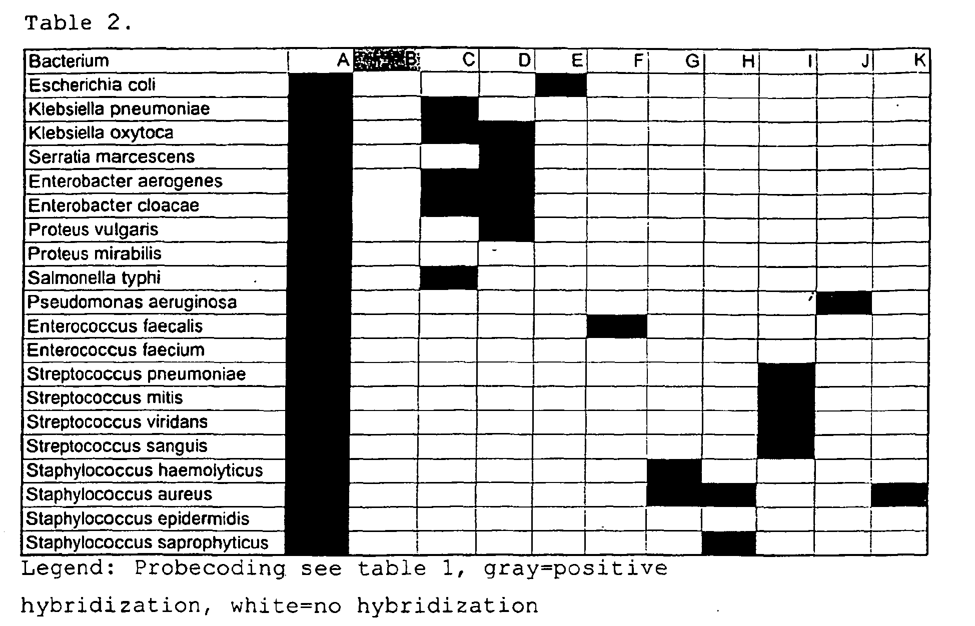

Testing a method in whole-blood samples.
Preliminary testing of a new method in 50 whole blood samples which were found positive upon pre-culturing yielded a correlation of 96% between a method described here and the classical culturing method which was also applied to each of the 50 samples.
However, the results of a method described here could be obtained within 3 hours while culturing results took a mean analysis-time of 32 hours.

### Further practical application

Septicemia is a pathological condition in which viable and multiplying bacteria may be present in the bloodstream.
This condition may occur after trauma or surgery (especially of the visceral organs), immunosuppression and obstetrical complications. It is a potential life-threatening condition and appropriate information on the causative agent should be available to the clinician as soon as possible. Bacteriological analysis of bloodsamples is routinely carried out by selective culturing of blood, which has previously been incubated on a general medium in a blood culture system. Currently, three continuous-reading, automated and computed blood culture systems are available in clinical microbiology today: the BacTec 9240 (Becton Dickinson Instruments), the BacT/Alert (BTA, Organon Teknika) and the Extra Sensing Power (Difco Laboratories). All of these machines measure the production-rate of a bacterium specific metabolite in the culture-bottle containing the patient's blood supplemented with general nutrient broth. Subsequently if bacterial metabolic activity is detected, the positive blood culture sample is plated on appropriate selective media for further analysis. Microbial identification, taking the positive blood culture as a starting point, takes 24 n to 72 h to complete. Reduction of the analysis time may result in reduction of the use of broad-spectrum antibiotics as the genus or species of a pathogen gives an indirect indication of the most appropriate antibiotic. Subsequently, this may result in a lower frequency of emergence of resistance against broad-spectrum antibiotics. Furthermore, it may result in lower cost because suppressive empiric therapy may be substituted by tailored and less expensive antibiotics with a smaller spectrum. Several methods for rapid detection of pathogens in human blood have been described previously, most of them using the polymerase chain reaction or fluorescently labeled probes. Although these methods are fast and accurate, routine bacteriological analysis still heavily relies on classical culturing technique. It was therefore decided that for a molecular biological method to be successfully implemented in routine bacteriology it should be fast (maximally 1-4 h) and very easy-to-use (e.g. as complex as the preparation of a Gram-stained slide). A simple and direct whole-cell hybridization assay using species- and genus specific, fluorescently labeled oligonucleotide probes, was developed and validated. The probes described in this further practical application section comprise of single strain oligonucleotides labelled with fluorescein iso-thiocyanate at the 5'end; complementary to a genus- or species-specific sequence on the 16S- or the 23S ribosomal RNA of the target-organism.

### Materials and methods

### Blood samples

During the time of this study a total of 182 blood samples which tested positive in the BactAlert-blood culture machine were processed simultaneously, using both whole-cell hybridization and accepted culturing methods.

### Culturing

Microorganisms cultivated from positive blood cultures were identified by using the API-testsystem (BioMerieux, France) or by using standard microbiological methods.

### Probes

The characteristics of the probes used in this study are listed in Table 3. All probes consist of a single stranded oligonucleotide sequence covalently linked with fluorescein iso-thiocyanate at the 5'-end. Probes were synthesized by EuroGentec BV (Maastricht, The Netherlands)

**Table 3. Oligonucleotide probes used for hybridization of some pathogens normally detected in blood from septic patients**

| **Probe*** | **Sequence (5'-->3')** | **Target(s)** | **Preferred antibiotic**** |
|---|---|---|---|
| EUB | GCTGCCTCCCGTAGGAGT | Bacterial Kingdom | n.a.*** |
| non-EUB | ACTCCTACGGGAGGCAGC | negative control | n.a. |
| STREP | GTTAGCCGTCCCTTTCTGG | *Streptococcus* spp. | Penicilin G |
| EFAEC | TTATCCCCCTCTGATGGG | *Enterococcus faecalis* | Amoxycillin |
| EFAEM | GCCACTCCTCTTTTTCCGG | *Enterococcus faecium* | Vancomycin |
| STAUR | AGAGAAGCAAGCTTCTCGTCCG | *Staphylococcus aureus* | *Flucloxacillin* |
| CNS | CGACGGCTAGCTCCAAATGGTTACT | Coagulase-negative | Vancomycin |
| | | Staphylococci | |
| ECOLI | GCAAAGGTATTAACTTTACTCCC | *Escherichia coli* | Amoxycillin |
| PSEUDAER | GGACGTTATCCCCCACTAT | *Pseudomonas aeruginosa* | Piperacillin+ |
| | | | aminoglycoside |
| ENTBAC | CATGAATCACAAAGTGGTAAGCGCC | *Enterobacterium spp* | 2^{nd} generation |
| | | | cephalosporin |

| | | | |
|---|---|---|---|
| * All probes consist of single strain DNA covalently linked with flurorescein iso-thyocyanate at the 5'-end. The E. coli-specific probe is directed against 23S rRNA, the other probes are directed against the 16S rRNA. Probe-nomenclature consists of mnemonics instead of a formal nomenclature-system for reasons of convenience. ** The antibiotic of first choice *** not applicable **** Limited choice from all available therapeutics. On the basis of the local epidemiological situations other therapeutics may prevail | | | |

### Whole cell hybridization

After gramstaining a streak-out preparation of a positive blood culture, a choice was made on the subsequent permeabilization-protocol and a set of appropriate probes (Table 3). Grampositive streptococci were permeabilized by incubating the fixed slide in a permeabilization-buffer (1 mg/ml lysozyme) during 5 min, gram-positive staphylococci were permeabilized by incubating the fixed slide in a second permeabilization-buffer (10 units/ml lysostaphin) during 20 min. Gram-negative rods were not permeabilized. Other gram-morphologies were not considered in this section because of the low incidence of these groups of bacteria in septicemia. From a positive blood culture fifteen µl was pipetted on a glass slide and subsequently streaked out. After air-drying the slide, the cells on the slide were fixed in a 4% formaldehyde-solution in pure ethanol. After permeabilization, the cells on the slide were hybridized at 50°C. Gram-negative rods were hybridized during 45 min, gram-positive staphylococci were hybridized during 2 h and gram-positive streptococci were hybridized during 5 min. Per gram-type a different set of probes was chosen (see Table 4).

**Table 4 Performance of the method**

| **Application-criterion*** | **Probe** | **Target organism** | **n**** | **r***** |
|---|---|---|---|---|
| Each assay | EUB | All bacteria | 182 | 1.00 |
| | non-EUB | negative control | 182 | 1.00 |
| Gram-positive chains | STREP | *Streptococcus spp.* | 20 | 1.00 |
| | EFAEC | *Enterococcus faecalis* | 10 | 1.00 |
| | EFAEM | *Enterococcus faecium* | 7 | 1.00 |
| Gram-positive clumps | STAUR | *Staphylococcus Aureus* | 13 | 1.00**** |
| | CNS | *Coagulase-negative* | 73 | 1.00 |
| | | *Staphylococci* | | |
| Gram-negative rods | ECOLI | *Escherichia coli* | 23 | 1.00 |
| | PSEUDAER | *Pseudomonas aeruginosa* | 4 | 1.00 |
| | ENTBAC | *Enerobacterium spp.* | 23 | 1.00 |

| | | | | |
|---|---|---|---|---|
| * The application criterion is the micromorphology of the pathogen in the initial gram-stain which is made after the BactAlert blood culture machine has detected microbolic activity in the bloodsample ** n=number of strains positively identified by culturing *** r=correlation coefficient. This is the number of matching identifications between FISH and traditional culturing divided by the total number of assays **** Comprises of 66% of the total number of samples tested. Other samples showed no signal with the positive control probe | | | | |

However, a probe (i.e. EUB-probe) positive for almost all bacteria and the reverse complementary probe (i.e. non-EUB probe) were included as a positive and negative control respectively, irrespective of the gram-type. Prior to use, probes were diluted to a concentration of 10 ng/ml in hybridization-buffer (20 mM Tris-HCl, 0.9M NaCl, 0.1% SDS, pH 7.2). After hybridization, the slides were rinsed during 10 min at 50°C in washing buffer (20 mM Tris-HCl, 0.9M NaCI, 0.1% SDS, pH 7.2) and mounted with VectaShield (Vector Laboratories, Burlinghame, USA). Immediately hereafter, the slides were evaluated using an epifluorescence microscope.

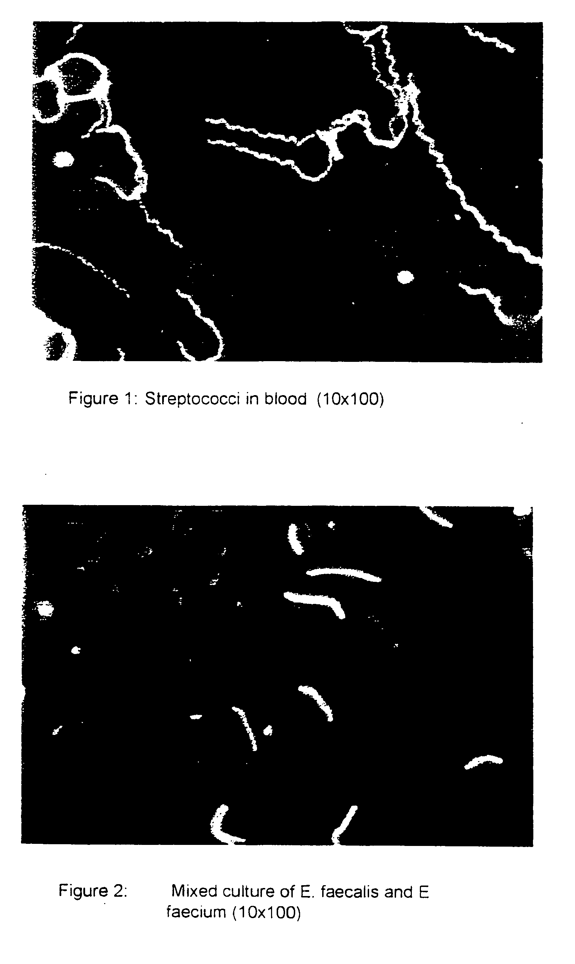

The results of this section show that identification using whole-cell hybridization dramatically increases the speed of the diagnosis. In Figure 1 a typical example of the microscopic image obtained after hybridizing a blood sample obtained from a patient suffering from *Streptococcus pneumoniae* sepsis using the STREP-probe is shown. Using the described protocol, a clear-cut positive signal was obtained. Repeated microscopic evaluation by, different observers confirmed the unambiguity of the interpretation of the images obtained by this method. In Table 4, the results of the study are listed. The observation that all strains hybridize positively with the EUB-probe indicates that the hybridization protocol is applicable for whole cell hybridization of the bacterial species and genera tested in this study. The negative results obtained with the non-EUB probe indicate the absence of aspecific interaction between the probe and constituents of the cellular matrix. The speed of diagnosis (after the sample is positive in the BactAlert blood culture machine) varies between 25 min (streptococci/enterococci) and 2 h (staphylococci), while routine bacteriological determination would take at least 24 h to 48 h. The advantage to the patient is obvious because, as can be read from Table 3, the clinician is able to start appropriate antimicrobial therapy within the working day instead of after 24 h to 48 h. Being able to choose the most appropriate antibiotic also diminishes the need for broad-spectrum antibiotics therewith indirectly lowering the incidence of antibiotic-resistance. In table 4 the results obtained in this section are mentioned.

### Broader application of the product

In its current form the product can be used for fast in situ detection of pathogenic bacteria in blood. However, the product can also be used for detection of pathogens in other clinical samples. Experiments using the current product (i.e. probes and protocol) in another type of clinical sample have been carried out. The sample-types were: liquor and ascites. Results indicate that application of the product in these samples is perfectly well possible. Future application include:
- New probes for other relevant species/genera
- Other types of clinical samples like: sputum, pus, urine, where generally non-septicaemic bacteria, such as Legionella pneumoniae can be found, using a method according to the invention.

### Advantages of whole-cell hybridization

- Rapid diagnosis enabling the clinician to easily choose the appropriate antibiotic
- Less use of broad spectrum antibiotics, therewith lowering the incidence of antibiotic resistance
- Cheap, a typical FISH-analysis is about 50% cheaper than a traditional culturing-based analysis.
- Easy to perform. A FISH-based analysis requires less actions and less hands-on time than a gram stain

### Lay-out of kits

There are many possibilities for possible kit formats, several are listed below.
One main kit for 100 Tests (1 test is 1 positive control, 1 negative control and one unknown) consisting of all kits 1-4 listed below including a detailed protocol. Or the kits listed below alone or in a combination.

### Kit 1 (the control kit):

- Lyophilized positive control probe (such as EUB or probe(s) functionally related thereto)
- Lyophilized negative control probe (such as non-EUB or probe(s) functionally related thereto)
- Lyophilized hybridization buffer (20 mM Tris-HCl, 0.9M NaCl, 0.1% SDS, pH 7.2)

### Kit 2 for gram-negative samples:

- Lyophilized probe such as ECOLI, PSEUDAER, ENTBAC or probe(s) functionally related thereto
- Lyophilized hybridization buffer (20 mM Tris-HCl, 0.9M NaCl, 0.1% SDS, pH 7.2)

### Kit 3 for gram-positive streptococci-like bacteria:

- Lyophilized probes such as STREP, EFAEC, EFAEM or probe(s) functionally related thereto
- Lyophilized hybridization buffer (20 mM Tris-HCI, 0.9M NaCL, 0.1% SDS, pH 7.2) with 1 mg/ml hen-eggwhite lysozyme.

### Kit 4 for staphylococci-like bacteria:

- Lyophilized probes such as STAUR, CNS or probe(s) functionally related thereto
- Lyophilized hybridization buffer (20 mM Tris-HC1, 0.9M NaCl, 0.1% SDS, pH 7.2) with 10 units/ml lysostaphin

Basic labeling of probes is with fluorescein iso-thio cyanate. Alternatively kits may contain probes with other fluorescent labels e.g. Cy-3, CyberGreen, Phyco-erythrin etc., or combinations therewith. Alternatively kits may contain a number of probes in one tube (Eppendorf cup) with different fluorescent labels for simultaneous detection of different targets with one test. We have successfully detected streptococci and enterococci in one sample in this way. In Fig 2 an example of this type of application is shown. Here, a mixed infection of morphologically indistinguishable gram-positive streptococcus-like bacteria are successfully hybridized with both the STREP-probe (FITC-label) and the EFAEC-probe (Cy3-label). Other combinations of the above kits may also be provided as one kit for a specific application.

### Legends

Figure 1: Cells of *Streptococcus pneumoniae* show intense bright fluorescence after min. of incubation with the STREP-probe at 50°C. Magnification = 10x100, fluorochrome = fluorescein iso-thiocyanate
Figure 2: Mixed infection with *E. faecalis* and *S. pneumoniae* hybridized simultaneously with both the STREP-probe (FITC-label) and the EFAECAL-probe (Cy3-label). Also a fluorescent DNA/RNA stain (DAPI) has been applied to detect all nucleic acid.

## Claims

1. A method for determining a bacterium suspected of being present in a sample comprising
a) testing said sample by Gram-staining and determining the rod or coccus character of said bacterium and when said Gram-staining indicates the presence of a Gram-positive bacterium with a coccus character, further determining a chain-like or clump-like character of said bacterium,
b) testing said sample with a nucleic acid probe according to an *in situ* hybridisation protocol selected on the basis of the outcome of said Gram-staining, wherein said protocol comprises;
(i) when said Gram-staining indicates the presence of a Gram-negative bacterium with a coccus character, subjecting said sample to a treatment with a lysis buffer comprising lysozyme, and
(ii) when said Gram-staining indicates the presence of a Gram-positive bacterium with a rod character, subjecting said sample to a treatment with a lysis buffer comprising lysozyme and/or Proteinase K, and
(iii) when said Gram-staining indicates the presence of a Gram-positive bacterium with a coccus and chain-like character subjecting said sample to a treatment with a lysis buffer comprising lysozyme and,
(iv) when said Gram-staining indicates the presence of a Gram-positive bacterium with a coccus and clump-like character subjecting said sample to a treatment with a lysis buffer comprising lysostaphin and/or Proteinase K.

2. A method according to claim 1 wherein said sample is a clinical sample.

3. A method according to claim 2 wherein said sample is mammalian blood, preferably being derived from a human.

4. A method according to claim 3, wherein said character is of the Gram-negative rod type, further comprising hybridising said sample with at least one probe selected from a group of probes capable of hybridising with nucleic acid found in *Escherichia coli,* in *Klebsiella pneumoniae,* in *Klebsiella oxytoca,* in *Serratia marcescens,* in *Enterobacter aerogenes,* in *Enterobacter cloacae,* in *Proteus vulgaris,* in *Proteus mirabilis,* in *Salmonella typhi,* in *Pseudomonas aeruginosa.*

5. A method according to claim 4 wherein said nucleic acid is ribosomal RNA.

6. A method according to claim 5 wherein said probe is having no more than five, preferably no more than two mismatches with a probe selected of a group composed of probes having a sequence GCCTGCCAGTTTCGAATG or GTAGCCCTACTCGTAAGG or GAGCAAAGGTATTAACTTTACTCCC or GTTAGCCGTCCCTTTCTGG.

7. A method according to claim 1, wherein said character is of the Gram-positive chain-like type further comprising hybridising said sample with at least one probe selected from a group of probes capable of hybridising with nucleic acid found in *Enterococcus faecalis,* in *Streptococcus pneumoniae,* in *Streptococcus mitis,* in *Streptococcus viridans,* in *Streptococcus sanguis,* in *Enterococcus faecium.*

8. A method according to claim 7, wherein said nucleic acid is ribosomal RNA.

9. A method according to claim 8 wherein said probe is having no more than five, preferably no more than two mismatches with a probe selected of a group composed of probes having a sequence TTATCCCCCTCTGATGGG or AGAGAAGCAAGCTTCTCGTCCG or GCCACTCCTCTTTTTCCGG.

10. A method according to claim 1, wherein said character is of the Gram-positive clump-like type further comprising hybridising said sample with at least one probe selected from a group of probes capable of hybridising with nucleic acid found in *Staphylococcus aureus,* in *Staphylococcus haemolyticus,* in *Staphylococcus-saprophyticus.*

11. A method according to claim 10 wherein said nucleic acid is ribosomal RNA.

12. A method according to claim 11 wherein said probe is having no more than five, preferably no more than two mismatches with a probe selected of a group composed of probes having a sequence GCTAATGCAGCGCGGATCC or CCGAAGGGGAAGGCTCTA or AGAGAAGCAAGCTTCTCGTCCGTT.

13. A method according to any of claims 1 to 12 further comprising hybridising said sample with at least one positive control nucleic acid probe and/or with at least one negative control nucleic acid probe.

14. A method according to claim 13 wherein said positive control probe comprises no more than five mismatches with a probe with the sequence GCTGCCTCCCGTAGGAGT and/or wherein said negative control probe comprises no more than five mismatches with a probe with the sequence ACTCCTACGGGAGGCAGC.

15. A method according to anyone of claims 1 to 14 further comprising a one-step procedure to bind bacteria present in said sample to a microscopic slide and simultaneously fix intracellular structures.

16. A method according to anyone of claims 1 to 15 wherein said probe is selected for its reactivity with one or a group of bacterial genera and/or species having congruent susceptibility to antibiotic treatment.

## Patentansprüche

1. Verfahren zur Bestimmung eines Bakteriums, von dem man annimmt, dass es in einer Probe vorhanden sein könnte, umfassend:
a) Testen der Probe durch Gram-Färbung und Bestimmen des Stäbchen- oder Kokkencharakters des Bakteriums, und wenn die Gram-Färbung die Anwesenheit eines Gram-positiven Bakteriums mit Kokkencharakter anzeigt, weiter Bestimmen des kettenartigen oder haufenartigen Charakters des Bakteriums;
b) Testen der Probe mit einer Nucleinsäuresonde gemäß einer in-situ-Hybridisierungsvorschrift, die auf der Basis des Ergebnisses der Gram-Färbung ausgewählt wird, wobei die Vorschrift Folgendes umfasst:
(i) wenn die Gram-Färbung die Anwesenheit eines Gram-negativen Bakteriums mit Kokkencharakter anzeigt, Behandeln der Probe mit einem Lysepuffer, der Lysozym umfasst; und
(ii) wenn die Gram-Färbung die Anwesenheit eines Gram-positiven Bakteriums mit Stäbchencharakter anzeigt, Behandeln der Probe mit einem Lysepuffer, der Lysozym und/oder Proteinase K umfasst; und
(iii) wenn die Gram-Färbung die Anwesenheit eines Gram-positiven Bakteriums mit Kokken- und kettenartigem Charakter anzeigt, Behandeln der Probe mit einem Lysepuffer, der Lysozym umfasst; und
(iv) wenn die Gram-Färbung die Anwesenheit eines Gram-positiven Bakteriums mit Kokken- und haufenartigem Charakter anzeigt, Behandeln der Probe mit einem Lysepuffer, der Lysostaphin und/oder Proteinase K umfasst.

2. Verfahren gemäß Anspruch 1, wobei die Probe eine klinische Probe ist.

3. Verfahren gemäß Anspruch 2, wobei es sich bei der Probe um Säugerblut handelt, das vorzugsweise von einem Menschen stammt.

4. Verfahren gemäß Anspruch 3, wobei der Charakter der Gram-negative Stäbchentyp ist, weiterhin umfassend das Hybridisieren der Probe mit wenigstens einer Sonde, die aus der Gruppe von Sonden ausgewählt ist, die mit Nucleinsäure hybridisieren können, die man in *Escherichia coli,* in *Klebsiella pneumoniae,* in *Klebsiella oxytoca,* in *Serratia marcescens,* in *Enterobacter aerogenes,* in *Enterobacter cloacae,* in *Proteus vulgaris,* in *Proteus mirabilis,* in *Salmonella typhi* oder in *Pseudomonas aeruginosa* findet.

5. Verfahren gemäß Anspruch 4, wobei es sich bei der Nucleinsäure um ribosomale RNA handelt.

6. Verfahren gemäß Anspruch 5, wobei die Sonde nicht mehr als fünf, vorzugsweise nicht mehr als zwei Fehlpaarungen mit einer Sonde aufweist, die aus der Gruppe ausgewählt ist, die aus Sonden mit der Sequenz GCCTGCCAGTTTCGAATG oder GTAGCCCTACTCGTAAGG oder GAGCAAAGGTATTAACTTTACTCCC oder GTTAGCCGTCCCTTTCTGG besteht.

7. Verfahren gemäß Anspruch 1 wobei der Charakter der Gram-positive kettenartige Typ ist, weiterhin umfassend das Hybridisieren der Probe mit wenigstens einer Sonde, die aus der Gruppe von Sonden ausgewählt ist, die mit Nucleinsäure hybridisieren können, die man in *Enterococcus faecalis,* in *Streptococcus pneumoniae,* in *Streptococcus mitis,* in *Streptococcus viridans,* in *Streptococcus sanguis* oder in *Enterococcus faecium* findet.

8. Verfahren gemäß Anspruch 7, wobei es sich bei der Nucleinsäure um ribosomale RNA handelt.

9. Verfahren gemäß Anspruch 8, wobei die Sonde nicht mehr als fünf, vorzugsweise nicht mehr als zwei Fehlpaarungen mit einer Sonde aufweist, die aus der Gruppe ausgewählt ist, die aus Sonden mit der Sequenz TTATCCCCCTCTGATGGG oder AGAGAAGCAAGCTTCTCGTCCG oder GCCACTCCTCTTTTTCCGG besteht.

10. Verfahren gemäß Anspruch 1, wobei der Charakter der Gram-positive haufenartige Typ ist, weiterhin umfassend das Hybridisieren der Probe mit wenigstens einer Sonde, die aus der Gruppe von Sonden ausgewählt ist, die mit Nucleinsäure hybridisieren können, die man in *Staphylococcus aureus,* in *Staphylococcus haemolyticus* oder in *Staphylococcus saprophyticus* findet.

11. Verfahren gemäß Anspruch 10, wobei es sich bei der Nucleinsäure um ribosomale RNA handelt.

12. Verfahren gemäß Anspruch 11, wobei die Sonde nicht mehr als fünf, vorzugsweise nicht mehr als zwei Fehlpaarungen mit einer Sonde aufweist, die aus der Gruppe ausgewählt ist, die aus Sonden mit der Sequenz GCTAATGCAGCGCGGATCC oder CCGAAGGGGAAGGCTCTA oder AGAGAAGCAAGCTTCTCGTCCGTT besteht.

13. Verfahren gemäß einem der Ansprüche 1 bis 12, weiterhin umfassend das Hybridisieren der Probe mit wenigstens einer positiven Kontrollnucleinsäuresonde und/oder mit wenigstens einer negativen Kontrollnucleinsäuresonde.

14. Verfahren gemäß Anspruch 13, wobei die positive Kontrollsonde nicht mehr als fünf Fehlpaarungen mit einer Sonde mit der Sequenz GCTGCCTCCCGTAGGAGT aufweist und/oder wobei die negative Kontrollsonde nicht mehr als fünf Fehlpaarungen mit einer Sonde mit der Sequenz ACTCCTACGGGAGGCAGC aufweist.

15. Verfahren gemäß einem der Ansprüche 1 bis 14, weiterhin umfassend ein einstufiges Verfahren zur Bindung von Bakterien, die in der Probe vorhanden sind, an einen Objektträger und gleichzeitigen Fixieren von intrazellulären Strukturen.

16. Verfahren gemäß einem der Ansprüche 1 bis 15, wobei die Sonde anhand ihrer Reaktivität gegenüber einer oder einer Gruppe von Bakteriengattungen und/oder -spezies, die eine kongruente Anfälligkeit für eine Antibiotikumbehandlung aufweisen, ausgewählt wird.

## Revendications

1. Procédé pour déterminer une bactérie suspectée d'être présente dans un échantillon comprenant
a) le test dudit échantillon par coloration de Gram et la détermination du caractère de bâtonnet ou de coque de ladite bactérie et quand ladite coloration de Gram indique la présence d'une bactérie Gram-positive ayant un caractère de coque, la détermination supplémentaire d'un caractère analogue à une chaîne ou analogue à un amas de ladite bactérie,
b) le test dudit échantillon avec une sonde d'acide nucléique selon un protocole d'hybridation *in situ* choisi sur la base du résultat de ladite coloration de Gram, où ledit protocole comprend :
(i) quand ladite coloration de Gram indique la présence d'une bactérie Gram-négative ayant un caractère de coque, l'exposition dudit échantillon à un traitement avec un tampon de lyse comprenant du lysozyme, et
(ii) quand ladite coloration de Gram indique la présence d'une bactérie Gram-positive ayant un caractère de bâtonnet, l'exposition dudit échantillon à un traitement avec un tampon de lyse comprenant du lysozyme et/ou de la Protéinase K, et
(iii) quand ladite coloration de Gram indique la présence d'une bactérie Gram-positive ayant un caractère de coque et analogue à une chaîne, l'exposition dudit échantillon à un traitement avec un tampon de lyse comprenant du lysozyme, et
(iv) quand ladite coloration de Gram indique la présence d'une bactérie Gram-positive ayant un caractère de coque et analogue à un amas, l'exposition dudit échantillon à un traitement avec un tampon de lyse comprenant de la lysostaphine et/ou de la Protéinase K.

2. Procédé selon la revendication 1 où ledit échantillon est un échantillon clinique.

3. Procédé selon la revendication 2 où ledit échantillon est du sang de mammifère, de préférence provenant d'un humain.

4. Procédé selon la revendication 3 où ledit caractère est de type bâtonnet Gram-négatif, comprenant en outre l'hybridation dudit échantillon avec au moins une sonde choisie dans un groupe de sondes capables de s'hybrider avec un acide nucléique trouvé dans *Escherichia coli,* dans *Klebsiella pneumoniae,* dans *Klebsiella oxytoca,* dans *Serratia marcescens,* dans *Enterobacter aerogenes,* dans *Enterobacter cloacae,* dans *Proteus vulgaris,* dans *Proteus mirabilis,* dans *Salmonella typhi,* dans *Pseudomonas aeruginosa.*

5. Procédé selon la revendication 4 où ledit acide nucléique est un ARN ribosomal.

6. Procédé selon la revendication 5 où ladite sonde n'a pas plus de cinq, de préférence pas plus de deux mésappariements avec une sonde choisie dans un groupe composé de sondes ayant une séquence GCCTGCCAGTTTCGAATG ou GTAGCCCTACTCGTAAGG ou GAGCAAAGGTATTAACTTTACTCCC ou GTTAGCCGTCCCTTTCTGG.

7. Procédé selon la revendication 1 où ledit caractère est de type analogue à une chaîne Gram-positif comprenant en outre l'hybridation dudit échantillon avec au moins une sonde choisie dans un groupe de sondes capables de s'hybrider avec un acide nucléique trouvé dans *Enterococcus faecalis,* dans *Streptococcus pneumoniae,* dans *Streptococcus mitis,* dans *Streptococcus viridans,* dans *Streptococcus sanguis,* dans *Enterococcus faecium.*

8. Procédé selon la revendication 7 où ledit acide nucléique est un ARN ribosomal.

9. Procédé selon la revendication 8 où ladite sonde n'a pas plus de cinq, de préférence pas plus de deux mésappariements avec une sonde choisie dans un groupe composé de sondes ayant une séquence TTATCCCCCTCTGATGGG ou AGAGAAGCAAGCTTCTCGTCCG ou GCCACTCCTCTTTTTCCGG.

10. Procédé selon la revendication 1 où ledit caractère est de type analogue à un amas Gram-positif comprenant en outre l'hybridation dudit échantillon avec au moins une sonde choisie dans un groupe de sondes capables de s'hybrider avec un acide nucléique trouvé dans *Staphylococcus aureus,* dans *Staphylococcus haemolyticus,* dans *Staphylococcus saprophyticus.*

11. Procédé selon la revendication 10 où ledit acide nucléique est un ARN ribosomal.

12. Procédé selon la revendication 11 où ladite sonde n'a pas plus de cinq, de préférence pas plus de deux mésappariements avec une sonde choisie dans un groupe composé de sondes ayant une séquence GCTAATGCAGCGCGGATCC ou CCGAAGGGGAAGGCTCTA ou AGAGAAGCAAGCTTCTCGTCCGTT.

13. Procédé selon l'une quelconque des revendications 1 à 12 comprenant en outre l'hybridation dudit échantillon avec au moins une sonde d'acide nucléique témoin positive et/ou avec au moins une sonde d'acide nucléique témoin négative.

14. Procédé selon la revendication 13 où ladite sonde témoin positive comprend au plus cinq mésappariements avec une sonde ayant la séquence GCTGCCTCCCGTAGGAGT et/ou où ladite sonde témoin négative comprend au plus cinq mésappariements avec une sonde ayant la séquence ACTCCTACGGGAGGCAGC.

15. Procédé selon l'une quelconque des revendications 1 à 14 comprenant en outre une procédure en une étape pour lier les bactéries présentes dans ledit échantillon à une lame de microscope et simultanément fixer les structures intracellulaires.

16. Procédé selon l'une quelconque des revendications 1 à 15 où ladite sonde est choisie pour sa réactivité avec un ou un groupe de genres bactériens et/ou d'espèces bactériennes ayant une sensibilité congruente à un traitement antibiotique.
